## Europäisches Patentamt
## European Patent Office
## Office européen des brevets

(19)

(11) Veröffentlichungsnummer: **0 017 832**
**B1**

(12) # EUROPÄISCHE PATENTSCHRIFT

(45) Veröffentlichungstag der Patentschrift:
**22.12.82**

(51) Int. Cl.³: **C 07 D 319/06**

(21) Anmeldenummer: **80101704.7**

(22) Anmeldetag: **31.03.80**

(54) **Verfahren zur Herstellung von 5-Brom-5-nitro-1,3-dioxan.**

(30) Priorität: **04.04.79 DE 2913466**

(43) Veröffentlichungstag der Anmeldung:
**29.10.80 Patentblatt 80/22**

(45) Bekanntmachung des Hinweises auf die Patenterteilung:
**22.12.82 Patentblatt 82/51**

(84) Benannte Vertragsstaaten:
**AT BE CH DE FR GB NL**

(56) Entgegenhaltungen:
**DE-A-2 263 206**
**DE-A-2 407 289**
**DE-B-1 920 298**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien, Postfach 1100 Henkelstrasse 67, D-4000 Düsseldorf 1 (DE)**

(72) Erfinder: **Gerhardt, Werner, Dr., Frans-Hals-Weg 7, D-4010 Hilden (DE)**

## Verfahren zur Herstellung von 5-Brom-5-nitro-1,3-dioxan

Die Erfindung betrifft ein verbessertes Verfahren zur Herstellung des antimikrobiell wirksamen 5-Brom-5-nitro-1,3-dioxans durch Acetalisierung von Formaldehyd mit 2-Brom-2-nitropropandiol-1,3 in Gegenwart von konzentrierter Schwefelsäure.

Nach der DE-B-19 20 298 kann 5-Brom-5-nitro-1,3-dioxan dadurch erhalten werden, dass man 2-Brom-2-nitropropandiol-(1,3) in Gegenwart von p-Toluolsulfonsäure und vorzugsweise Polyphosphorsäure als saure Katalysatoren acetalisiert, wobei die Umsetzung in Benzol oder in Abwesenheit von Lösungsmitteln durchgeführt wird. 5-Brom-5-nitro-1,3-dioxan fällt dabei in stark verunreinigter Form an und muss mittels aufwendiger Verfahren wie Wasserdampfdestillation oder Sublimation im Hochvakuum gereinigt werden.

Nach der DE-A-22 63 206 kann 5-Brom-5-nitro-1,3-dioxan durch Acetalisierung von Formaldehyd mit 2-Brom-2-nitropropandiol-(1,3) in Gegenwart von konzentrierter Schwefelsäure hergestellt werden, wobei man Ethylenchlorid als inertes Lösungsmittel benutzt. Dieses Verfahren liefert reinere Produkte als die in der DE-B-19 20 298 beschriebene Arbeitsweise. Es hat sich jedoch gezeigt, dass die Verfahrensprodukte in den meisten Fällen aus Lösungsmitteln wie wässrigem Methanol umkristallisiert werden müssen, um den gestellten Reinheitsanforderungen zu genügen. Überdies bedingt das Verfahren der DE-A-22 63 206 wegen der toxikologischen Bedenklichkeit des Ethylenchlorids besondere Sicherheitsvorkehrungen.

Es wurde nun ein Verfahren gefunden, das auf direktem Weg zu sehr reinem 5-Brom-5-nitro-1,3-dioxan führt und das weiterhin die Verwendung des toxikologisch bedenklichen Ethylenchlorids umgeht.

Gegenstand der Erfindung ist ein Verfahren zur Herstellung von 5-Brom-5-nitro-1,3-dioxan durch Umsetzung von 2-Brom-2-nitropropandiol-1,3 mit Formaldehyd bzw. Paraformaldehyd in einem inerten Lösungsmittel bei erhöhter Temperatur und in Gegenwart von konzentrierter Schwefelsäure, das dadurch gekennzeichnet ist, dass man als Lösungsmittel Tetrachlorethylen verwendet.

Es hat sich als zweckmässig erwiesen, das erfindungsgemässe Verfahren mit relativ geringen Lösungsmittelmengen durchzuführen. Man verwendet deshalb vorzugsweise auf 1 Kilogramm umzusetzendes 2-Brom-2-nitropropandiol-(1,3) 2 bis 4 Liter Tetrachlorethylen.

2-Brom-2-nitropropandiol und Formaldehyd bzw. Paraformaldehyd können in stöchiometrischen Mengen, das heisst im Molverhältnis 1 : 1 miteinander umgesetzt werden. Es ist jedoch von Vorteil, die Reaktion mit einem Aldehydüberschuss in der Grössenordnung von 10 bis 40% ablaufen zu lassen.

Das erfindungsgemässe Verfahren kann mit katalytischen Mengen Schwefelsäure durchgeführt werden. In diesem Fall muss das bei der Reaktion entstehende Wasser bei Rückflusstemperatur, zweckmässigerweise mit Hilfe eines Wasserabscheiders, laufend aus dem Reaktionsgemisch entfernt werden. Das bei der Reaktion entstehende Wasser kann aber auch durch Zugabe einer ausreichenden Menge konzentrierter Schwefelsäure gebunden werden.

Bei der praktischen Durchführung des Verfahrens gibt man 2-Brom-2-nitroprorandiol-(1,3) und Paraformaldehyd zu der vorgesehenen Menge Tetrachlorethylen, erhitzt die Mischung auf mindestens 70°C und gibt dann unter kräftigem Rühren langsam die konzentrierte Schwefelsäure zu. Anschliessend wird das Gemisch auf Rückflusstemperatur erhitzt. Bei der Verwendung katalytischer Mengen Schwefelsäure muss das Erhitzen so lange fortgesetzt werden, bis die berechnete Menge Reaktionswasser ausgeschleppt ist. Wendet man eine zur Bindung des Reaktionswassers ausreichende Menge konzentrierter Schwefelsäure an, so ist die Umsetzung in der Regel beendet, wenn die Mischung etwa 2 Stunden lang auf 80 bis 90°C erhitzt wurde.

Da es sich bei den anfallenden Reaktionsgemischen um relativ konzentrierte Lösungen handelt, nimmt man die Aufarbeitung zweckmässigerweise bei erhöhter Temperatur vor. In der Regel werden die Reaktionsgemische nur bis auf 50 bis 60°C abgekühlt; gegebenenfalls vorhandene grössere Mengen Schwefelsäure werden als untere Phase abgetrennt. Die organische Phase wird dann mehrmals mit Wasser, verdünnter Natronlauge und wieder mit Wasser gewaschen, wobei jedoch auf 50 bis 60°C vorgewärmte Waschflüssigkeiten verwendet werden. Aus den so behandelten Tetrachlorethylenlösungen fällt das reine, kristalline 5-Brom-5-nitro-1,3-dioxan nach dem Abkühlen auf 0°C aus.

Ein wesentlicher Vorteil des erfindungsgemässen Verfahrens gegenüber den Verfahren des Standes der Technik besteht darin, dass das 5-Brom-5-nitro-1,3-dioxan direkt aus dem als Reaktionsmedium verwendeten Tetrachlorethylen mit der in der Praxis geforderten hohen Reinheit ausfällt, wodurch die Reinigung durch Umkristallisieren aus einem zweiten Lösungsmittel entfällt. Weiterhin zeichnet sich das erfindungsgemässe Verfahren dadurch aus, dass es mit erheblich verkürzten Reaktionszeiten arbeit und dass es ohne Verwendung des toxikologisch bedenklichen 1,2-Dichlorethans durchgeführt werden kann.

Das nachfolgende Beispiel soll den Gegenstand der Erfindung näher erläutern, ihn jedoch nicht hierauf beschränken.

Beispiel

50 g (0,25 Mol) 2-Brom-2-nitropropandiol-(1,3), 9 g (0,3 Mol) Paraformaldehyd und 125 ml Tetrachlorethylen wurden unter Rühren auf 80°C erwärmt. Nach Zugabe von 0,3 ml konzentrierter Schwefelsäure wurde das .Gemisch 90 Minuten lang zum Rückflusskochen erhitzt, das heisst so lange bis sich im Wasserabscheider die berechnete Menge Reaktionswasser angesammelt hatte. Nach Abkühlung auf 55-60°C wurde die Reaktionsmischung nacheinander mit Wasser (zweimal 50 ml), 1 gew.%iger NaOH-Lösung (zweimal 50 ml) und Wasser (zweimal 50 ml) gewaschen, wobei jeweils auf 55-60°C erwärmte Waschflüssigkeiten verwendet wurden. Anschliessend wurde die Tetrachlorethylenlösung filtriert und auf 0°C abgekült. Die ausgefallenen farblosen Kristalle wurden abfiltriert und an der Luft getrocknet. Es wurden 36 g 5-Brom-5-nitro-1,3-dioxan (68% der Theorie) mit Schmelzpunkt 58 bis 60°C erhalten. Durch Einengen der Mutterlauge wurden weitere 8 g des Produktes (15% der Theorie) erhalten.

### Patentansprüche

1. Verfahren zur Herstellung von 5-Brom-5-nitro-1,3-dioxan durch Umsetzung von 2-Brom-2-nitro-propandiol-1,3 mit Formaldehyd oder Paraformaldehyd in einem inerten Lösungsmittel bei erhöhter Temperatur und in Gegenwart von konzentrierter Schwefelsäure, dadurch gekennzeichnet, dass man als Lösungsmittel Tetrachlorethylen verwendet.

2. Verfahren nach Anspruch 1, dadurch gekennzeichnet, dass man auf 1 Kilogramm umzusetzendes 2-Brom-2-nitropropandiol-(1,3) 2 bis 4 Liter Tetrachlorethylen verwendet.

### Claims

1. A process for the production of 5-bromo-5-nitro-1,3-dioxane by reacting 2-bromo-2-nitro-1,3-propane diol with formaldehyde or paraformaldehyde in an inert solvent at elevated temperature and in the presence of concentrated sulfuric acid, characterised in that tetrachloroethylene is used as the solvent.

2. A process as claimed in Claim 1, characterised in that 2 to 4 litres of tetrachloroethylene are used per kilogram of 2-bromo-2-nitro-1,3-propane diol to be reacted.

### Revendications

1. Procédé de préparation de 5-bromo-5-nitro-1,3-dioxanne par réaction du 2-bromo-2-nitro-propane diol-1,3 avec la formaldéhyde ou paraformaldéhyde dans un solvant inerte à température élevée et en présence d'acide sulfurique concentré, caractérisé en ce qu'on utilise comme solvant du tétrachloréthylène.

2. Procédé selon la revendication 1, caractérisé en ce que par kilogramme de 2-bromo-2-nitropropane diol-1,3 que l'on fait réagir on utilise 2 à 4 litres de tétrachloréthylène.